# EUROPÄISCHE PATENTSCHRIFT

(11) **EP 2 114 328 B1**
(45) Veröffentlichungstag und Bekanntmachung des Hinweises auf die Patenterteilung: **11.03.2015**
(21) Anmeldenummer: 08708227.7
(22) Anmeldetag: 25.01.2008
(51) Int. Cl.: A61F 9/007

(54) **AUGENIMPLANTAT**
OCULAR IMPLANT
IMPLANT OCULAIRE

(30) Priorität: 25.01.2007 DE 102007004906
(43) Veröffentlichungstag der Anmeldung: 11.11.2009
(73) Patentinhaber: Universität Rostock, 18055 Rostock (DE)
(72) Erfinder: SCHMIDT, Wolfram, 18109 Rostock (DE); STERNBERG, Katrin, 18055 Rostock (DE); BEHREND, Detlef, 18119 Rostock (DE); GUTHOFF, Rudolf, 18119 Rostock (DE); SCHMITZ, Klaus-Peter, 18119 Rostock (DE)
(74) Vertreter: Gulde & Partner
(86) Internationale Anmeldenummer: PCT/EP2008/050903
(87) Internationale Veröffentlichungsnummer: WO 2008/090225

(56) Entgegenhaltungen:
- WO-A-03/099175
- US-A- 5 338 291
- US-A1- 2003 135 149

## Beschreibung

Die vorliegende Erfindung betrifft ein Augenimplantat, insbesondere einen Glaukomstent mit den in Patentanspruch 1 genannten Merkmalen.

Die vorliegende Erfindung gehört zum Gebiet der Medizintechnik und stellt eine Verbesserung einer bestehenden Behandlungsmethode von Glaukomen dar. Ein Glaukom, umgangssprachlich auch als "Grüner Star" bezeichnet, beschreibt eine Gruppe von Augenkrankheiten, die gekennzeichnet sind durch einen zeitweise oder dauernd erhöhten Augeninnendruck, der die Blutversorgung des Sehnervs behindert. Bei lang andauernder Unterversorgung nimmt der Sehnerv Schaden und kann in Extremfällen absterben. Das Glaukom zählt zu den häufigsten Erblindungsursachen, vor allem weil es aufgrund seines häufig unauffälligen Krankheitsverlaufs oft zu lange unerkannt und damit unbehandelt bleibt. Man geht davon aus, dass es in Deutschland zusätzlich zu den offiziell Erkrankten (rund eine Million) noch einmal so viele Betroffene gibt. Etwa 10 % davon droht die Erblindung.

Es werden verschiedene Formen von Glaukomen unterschieden, vornehmlich primäre und sekundäre, wobei letztere eher als Begleiterscheinungen von anderen Krankheiten auftreten, bzw. nach anatomischen Merkmalen, Eng- und Offenwinkelglaukome. Diese Bezeichnungen beziehen sich auf den Winkel zwischen Hornhautrückfläche und Irisvorderfläche. Allen Glaukomen gemein ist, dass aufgrund verschiedener Ursachen das Kammerwasser, eine spezielle Flüssigkeit, welche Linse und Hornhaut mit Nährstoffen versorgt und zugleich Stoffwechselendprodukte heraustransportiert, nicht ausreichend abfließen kann. Da das Kammerwasser ständig neu gebildet wird, führt der behinderte Abfluss dazu, dass sich der Druck im geschlossenen System *Auge* erhöht.

Am häufigsten, mit ca. 80 - 90 %, sind sogenannte primäre Offenwinkelglaukome. Bei einem Offenwinkelglaukom kommt es zu einer Abflussbehinderung direkt im Abflussbereich des Kammerwinkels, im so genannten Trabekelmaschenwerk. Das Trabekelmaschenwerk, ein Filtersystem im Winkel zwischen Hornhaut und Regenbogenhaut (Kammerwinkel), besteht aus feinen Poren Da deren Größe aus bisher unbekannten Gründen vermindert ist, kann der Abfluss des Kammerwassers nicht, wie bei einem gesunden Auge, über das Trabekelmaschenwerk und weiter durch den sogenannten Schlemm-Kanal in die episkleralen Venen erfolgen.

Bei der Therapie von Glaukomen unterscheidet man zunächst zwischen medikamentösen Therapien, Laserbehandlungen und operativen Methoden. Letztere, in welche sich auch die vorliegende Erfindung einordnet, kommen erst zum Einsatz, wenn die ersten beiden nicht erfolgreich waren, und zielen darauf ab, chirurgisch eine Abflussmöglichkeit für das Kammerwasser zu schaffen. Dies geschieht bei Offenwinkelglaukomen in der Regel durch eine Trabekulektomie. Dabei wird ein Kanal geschaffen, durch den das Kammerwasser aus der Vorderkammer unter die Bindehaut in ein sogenanntes Sickerkissen (auch Filterkissen genannt) abfließen kann. Da es sich um Wunden handelt und sich naturgemäß die geschaffene Öffnung durch Fibrosen (exzessive Kollagenfaser-/Bindegewebsvermehrung) wieder verschließen kann, ist der Erfolg einer solchen Operation oft nicht dauerhaft. Nach gescheiterter Trabekulektomie, in den USA häufig auch schon früher, kommen alloplastische Implantate, sogenannte Drainagesysteme bzw. -implantate (Stents) zum Einsatz.

Typischerweise bestehen Glaukomimplantate aus einem Silikonelastomerschlauch, der Kammerwasser aus dem Augeninneren zu einer Drainageplatte aus unterschiedlichem Material leitet. Um diese entsteht eine unterschiedlich dicke Bindegewebsstruktur, durch die Kammerwasser in der Umgebung diffundiert. Je nach Ausgangssituation und Wundheilungsreaktion kann durch Implantate der intraokulare Druck in 50-100% der Fälle für viele Jahre kontrolliert werden. Dennoch treten bei der Implantation eines Drainagesystems Probleme auf, da durch den unkontrollierten Abfluss des Kammerwassers sogenannte Hypotonien (zu niedriger Augeninnendruck) auftreten können, die zu einer Aderhautabhebung bis zur Blutung sowie durch eine kollabierte Vorderkammer zu einer Hornhautdekompensation führen können. Um den Abfluss zu regulieren, wird der Querschnitt der Stents den Druckverhältnissen zum Zeitpunkt der Implantation angepasst. Mit im Rahmen der Wundheilung einsetzender Fibrose erhöht sich in der Regel der Flusswiderstand, so dass sich die eingangs angestrebte Flussrate verringern und das beabsichtigte Gleichgewicht wieder gestört werden kann. In diesen Fällen wird bisher ein erneuter Eingriff notwendig.

Zur Verhinderung von Komplikationen durch Hypotonien sind Implantate (US 5,041,081; US 7,025,740 B2 und US 5,127,901) mit einem Ventilmechanismus bekannt, der den Kammerwasserabfluss limitieren soll. Hierbei handelt es sich ausschließlich um Rückschlagventilmechanismen, die den Durchgang des Kammerwassers in einer Strömungsrichtung selbsttätig sperren. Ist der Druck im Augeninneren zu hoch, wird das Ventil geöffnet, bietet also einen gewissen Widerstand im Vergleich zu einem unkontrollierten Abfluss. Im Prinzip kann dieser Mechanismus aber nur den Rückfluss des Kammerwassers wirklich verhindern, der physikalisch im Normalfall ohnehin ausgeschlossen ist. Dafür müsste der Druck außerhalb höher sein als im Auge, was zum Beispiel im Falle des Tiefseetauschens auftreten könnte. Die vorgenannten Lösungen bieten keinen Schutz davor, dass sich der Flusswiderstand - beispielsweise durch Fibrose - mit der Zeit erhöht.

Die Druckschrift WO 03/099175 A1 offenbart ein röhrchenförmiges Augenimplantat mit einem durch eine Wandfläche begrenzten beidseitig offenen Hohlkanal in den Augenkammerwasser einströmen und wieder abfließen kann. Im Bereich der Wandfläche ist ebenfalls ein druckgesteuertes Ventil angeordnet.

Es ist die Aufgabe der vorliegenden Erfindung, ein Augenimplantat (Glaukomstent) anzugeben, welches einerseits den Augeninnendruck regulieren, d.h. auf einem gewünschten Niveau halten kann und andererseits verhindert, dass sich der Flusswiderstand - beispielsweise durch Fibrose - mit der Zeit erhöht. Ausgangspunkt der Erfindung ist ein Augenimplantat, welches ein Röhrchen aufweist, dessen Wandfläche einen Hohlkanal einschließt, der beidseitig in Längserstreckung des Hohlkanals offen ausgebildet ist, wobei eine erste Öffnung zum Einströmen von Augenkammerwasser und eine zweite Öffnung zum Abfließen des Augenkammerwassers vorgesehen ist, und wobei die Wandfläche durch ein flüssigkeitsdichtes Material ausgebildet ist, wobei im Bereich der Wandfläche mindestens ein druckgesteuertes Ventil angeordnet ist.

Erfindungsgemäß ist vorgesehen, dass das druckgesteuerte Ventil ein Membranventil ist, welches durch eine Basis und eine umlaufende Durchtrennung der Wandfläche ausgebildet ist, wobei die Wandfläche im Bereich der Basis nicht durchtrennt ist.

Zweckmäßige Ausgestaltungen der Erfindung sind in den Unteransprüchen enthalten.

Der erfindungsgemäße Glaukomstent enthält druckgesteuerte Ventile (Membranventile), die durch einen erhöhten Augeninnendruck geöffnet und bei wieder absinkendem Druck geschlossen werden, wobei diese druckgesteuerte Ventile in der Stentwand (und nicht innerhalb des Stents) angebracht sind. Damit kann der Abfluss reguliert und eine jederzeit optimal angepasste Flussrate erreicht werden. Denn die in der Stentwand angeordneten, druckgesteuerten Ventile ermöglichen zusätzlich den Abfluss des sich an den Stentaußenwänden sammelnden Kammerwassers. Selbst bei allmählicher Stenosierung des Stents kann hier der Abfluss gewährleistet werden, da eine neue Eintrittsmöglichkeit des Kammerwassers zur Verfügung steht, die aber nur dann Kammerwasser ableitet, wenn der Augeninnendruck entsprechend hoch ist. Somit wurde eine Lösung geschaffen, sowohl bei verändertem Augeninnendruck (im Vergleich zum Implantationszeitpunkt) als auch bei erhöhtem Flusswiderstand einen optimal angepassten Kammerwasserabfluss zu gewährleisten und einen konstanten Augeninnendruck zu erzielen.

Dazu weist das erfindungsgemäße Augenimplantat ein Röhrchen auf, dessen Wandfläche einen Hohlkanal einschließt, der beidseitig in Längserstreckung des Hohlkanals offen ausgebildet ist, wobei eine erste Öffnung zum Einströmen von Augenkammerwasser und eine zweite Öffnung zum Abfließen des Augenkammerwassers vorgesehen ist, und wobei die Wandfläche durch ein flüssigkeitsdichtes Material ausgebildet ist, und wobei im Bereich der Wandfläche mindestens ein druckgesteuertes Ventil angeordnet ist.

Es ist bevorzugt, dass das mindestens eine druckgesteuerte Ventil im Bereich der Wandfläche, jedoch nicht im Bereich der ersten Öffnung (zum Einströmen von Augenkammerwasser), d.h. nicht im (die erste Öffnung ausbildenden bzw. an die erste Öffnung angrenzenden) Randbereich der Wandfläche angeordnet ist. Dadurch wird neben der ersten Öffnung zum Einströmen von Augenkammerwasser mindestens eine weitere Öffnung zum Einströmen von Augenkammerwasser vorgesehen, die jedoch erst ab einem bestimmten Druck öffnet.

Vorzugsweise sind das Wandmaterial, die Wandstärke und die Wandstärkenverteilung über die Länge des Röhrchens derart gewählt, dass das Röhrchen nach Einbringen in die Vorderkammer des Auges derart in dieser platziert verbleibt, dass sich die erste Öffnung in der vorderen Augenkammer und die zweite Öffnung im subkonjunktivalen Raum befindet. Ebenfalls vorzugsweise sind das Wandmaterial, die Wandstärke und die Wandstärkenverteilung über die Länge des Röhrchens derart gewählt, dass das Röhrchen nach Einbringen in die Vorderkammer des Auges derart in dieser platziert verbleibt, dass sich die erste Öffnung in der vorderen Augenkammer und die zweite Öffnung im Uveoskleralraum befindet.

Vorzugsweise weist der innenliegende Hohlkanal des Röhrchens einen Durchmesser zwischen 50 µm und 1000 µm auf. Vorzugsweise weist der Hohlkanal entlang seiner Längserstreckung einen minimalen Innendurchmesser zwischen 50 µm und 80 µm auf. Vorzugsweise weist der Hohlkanal entlang seiner Längserstreckung einen maximalen Innendurchmesser zwischen 300 µm und 1000 µm, bevorzugt zwischen 350 µm und 400 µm auf. Vorzugsweise weist der Hohlkanal eine durch eine Blende ausgebildete Flussbegrenzung auf, wobei die Blende eine Öffnung aufweist, deren Fläche zwischen 1000 und 3000 µm², besonders bevorzugt zwischen 1500 und 2000 µm² entspricht. Vorzugsweise weist die Blende eine kreisrunde Öffnung mit einem Durchmesser zwischen 35 µm und 60 µm, besonders bevorzugt zwischen 40 µm und 50 µm auf. Vorzugsweise ist die Blende in einem Abstand von der ersten Öffnung angeordnet, der zwischen 0% und 10 % der Längserstreckung des Röhrchens entspricht. Besonders bevorzugt ist die Blende direkt an der ersten Öffnung angeordnet. Durch die bevorzugten Dimensionen des Röhrchendurchmessers und die Blende kann der Abfluss des erfindungsgemäßen Stents so eingestellt werden, dass der Augeninnendruck reguliert werden kann. Der Röhrchendurchmesser kann so groß gewählt werden, dass eine Erhöhung des Flusswiderstandes innerhalb des Hohlkanals - beispielsweise durch Fibrose - nicht erfolgt. Zwar kann eine Erhöhung des Flusswiderstandes im Bereich der (in ihrem Durchmesser deutlich kleineren) Blende - beispielsweise durch Fibrose - erfolgen, jedoch steht durch die in der Seitenwand des Röhrchens angeordneten druckgesteuerten Ventile eine neue, zusätzliche Eintrittsmöglichkeit des Kammerwassers zur Verfügung, die das "Sich Zusetzen" der Flussbegrenzung (Blende) - beispielsweise durch Fibrose - kompensieren können.

Vorzugsweise weist das Röhrchen eine Länge zwischen 3 mm und 20 mm auf. Vorzugsweise weist das Röhrchen eine Wandstärke zwischen 10 µm und 200 µm auf. Vorzugsweise weist das Röhrchen entlang seiner Längserstreckung eine minimale Wandstärke zwischen 10 µm und 20 µm auf. Vorzugsweise weist das Röhrchen entlang seiner Längserstreckung eine maximale Wandstärke zwischen 80 µm und 100 µm auf. Vorzugsweise erstreckt sich der Hohlkanal des Röhrchens über die volle Länge zwischen den beiden Enden des Röhrchens.

Vorzugsweise ist die erste Öffnung unmittelbar am ersten Ende des Röhrchens und die zweite Öffnung unmittelbar am zweiten Ende des Röhrchens angeordnet. Vorzugsweise weist das Röhrchen eine homogene Wandstärke auf. Vorzugsweise bildet das Röhrchen einen geraden Hohlzylinder oder einen schiefen Hohlzylinder aus. Vorzugsweise ist das Röhrchen aus einem flexiblen Material, einem Metall oder einer Metalllegierung ausgebildet. Vorzugsweise ist die Wandfläche des Röhrchens aus einem flexiblen Material, bevorzugt aus einem Elastomer, ausgebildet. Alternativ ist die Wandfläche des Röhrchens aus einem Metall oder einer Metalllegierung ausgebildet. Besonders bevorzugt ist die Wandfläche des Röhrchens aus einer Formgedächtnislegierung (NiTi) oder dem Edelstahl 316L ausgebildet.

Vorzugsweise sind die erste Öffnung und/oder die zweite Öffnung ringförmig ausgebildet. Das Ventil ist als Membranventil und bevorzugt als Zungenmembranventil ausgebildet. Das Membranventil ist durch eine Basis und eine umlaufende Durchtrennung der Wandfläche ausgebildet, wobei die Wandfläche im Bereich der Basis nicht durchtrennt ist. Vorzugsweise ist die Wandstärke der Wandfläche im Bereich der Basis geringer ist als die Wandstärke der Wandfläche im angrenzenden Bereich außerhalb des Ventils. Vorzugsweise beträgt die Wandstärke der Wandfläche im Bereich der Basis zwischen 30 und 80 % der Wandstärke der Wandfläche im angrenzenden Bereich außerhalb des Ventils. Eine reduzierte Wandstärke der Wandfläche erlaubt ein einfaches Öffnen des Ventils bei erhöhtem Augeninnendruck. Die Wandstärke der Wandfläche ist dabei so bemessen, dass das druckgesteuerte Ventil ab einem Druck von 10 mm Hg-Säule zu öffnen beginnt, ab einem Druck von 20 mm Hg-Säule voll geöffnet ist und bei einem Druck zwischen 10 mm bis 20 mm Hg-Säule kontinuierlich öffnet.

Vorzugsweise ist die umlaufende Durchtrennung durch einen Spalt in der Wandfläche des Röhrchens ausgebildet, wobei der Spalt eine Breite zwischen 2 µm und 20 µm aufweist. Vorzugsweise weist die Membran des Membranventils oder die Membran des Zungenmembranventils mindestens eine Öffnung auf, wobei die Öffnung (8) eine Fläche zwischen 80 und 200µm oder zwischen 500 und 2000 µm² aufweist. Durch diese Öffnung wird auch im geschlossenen Zustand des Ventils ein minimaler Fluss ermöglicht, der den Wiederverschluss behindert.

Vorzugsweise ist die Basis des Ventils zur ersten Öffnung ausgerichtet und die umlaufende Durchtrennung von der Basis in Richtung der zweiten Öffnung ausgerichtet. Vorzugsweise weist das Ventil eine Fläche auf, die zwischen 5 % und 10 % der Fläche der Wandfläche des Röhrchens entspricht. Vorzugsweise weist das Ventil eine axiale Längsausdehnung entlang der Längsachse des Röhrchens von maximal 15 % der Länge des Röhrchens und eine radiale Querausdehnung von maximal 15 % des Umfanges des Röhrchens auf.

Vorzugsweise sind eine Vielzahl von Ventilen (umlaufend angeordnet) vorgesehen. Vorzugsweise sind zwischen 2 und 8 Ventile vorgesehen. Vorzugsweise sind in radialer Richtung maximal 4 Ventile nebeneinander in der Längsrichtung zueinander versetzt und in axialer Richtung maximal 2 Ventile hintereinander angeordnet. Vorzugsweise weisen die Ventile zusammen eine Fläche auf, die zwischen 10 % und 35 % der Fläche der Wandfläche des Röhrchens entspricht. Durch die Anordnung mehrerer Ventile kann ein möglichst großes, zusätzliches Abflussgebiet bei ausreichender Festigkeit des Stents geschaffen werden. Außerdem sind die Ventile so dimensioniert, dass sie eine hohe Zuverlässigkeit aufweisen.

Der Stent befindet sich vorzugsweise bis 35 % seiner Länge in der vorderen Augenkammer. Vorzugsweise sind die Ventile in einem Abstand von der ersten Öffnung angeordnet sind, der zwischen 0 % und 35 % der Längserstreckung des Röhrchens entspricht. Vorzugsweise sind die Ventile in einem Abstand von der ersten Öffnung angeordnet sind, der zwischen 5 % und 35 % der Längserstreckung des Röhrchens entspricht. Entsprechend ist es vorzugsweise vorgesehen, dass sich keine Ventile auf der Mantelfläche in einem Abstand von der ersten Öffnung vorgesehen sind, der zwischen 35 % und 100 % der Längserstreckung des Röhrchens entspricht.

Die Erfindung soll nachstehend anhand von in den Figuren dargestellten Alusführungsbeispielen näher erläutert werden. Es zeigen:
- Figur 1: ein erfindungsgemäßes Augenimplantat in perspektivischer Ansicht,
- Figur 2: ein erfindungsgemäßes Augenimplantat in Aufsicht,
- Figur 3: eine Schnittdarstellung des in Figur 2 gezeigten Augenimplantats entlang der Linie X-X',
- Figur 4: ein erfindungsgemäßes Augenimplantat mit einer Vielzahl von druckgesteuerten Ventilen in perspektivischer Ansicht,
- Figur 5 a): ein in der Wandfläche des Stents angeordnetes, druckgesteuertes Ventil (in halb geöffneter Position) in schematischer, geschnittener Darstellung,

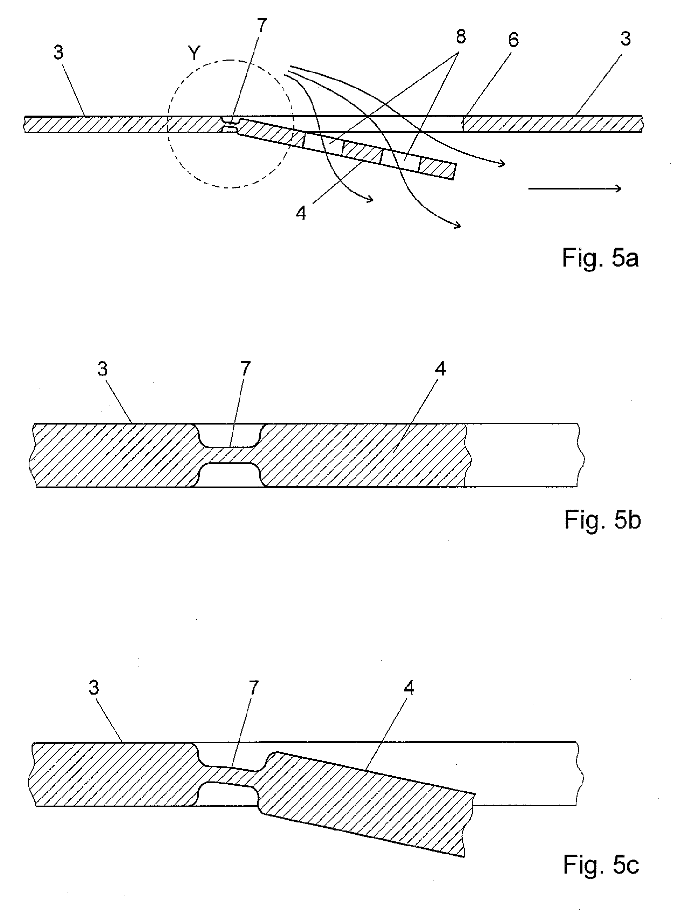

- Figur 5 b): eine vergrößerte Darstellung des in Figur 5a markierten Bereichs Y, wobei sich das druckgesteuerte Ventil im geschlossenen Zustand befindet,
- Figur 5 c): eine vergrößerte Darstellung des in Figur 5a markierten Bereichs Y, wobei sich das druckgesteuerte Ventil im geöffneten Zustand befindet,

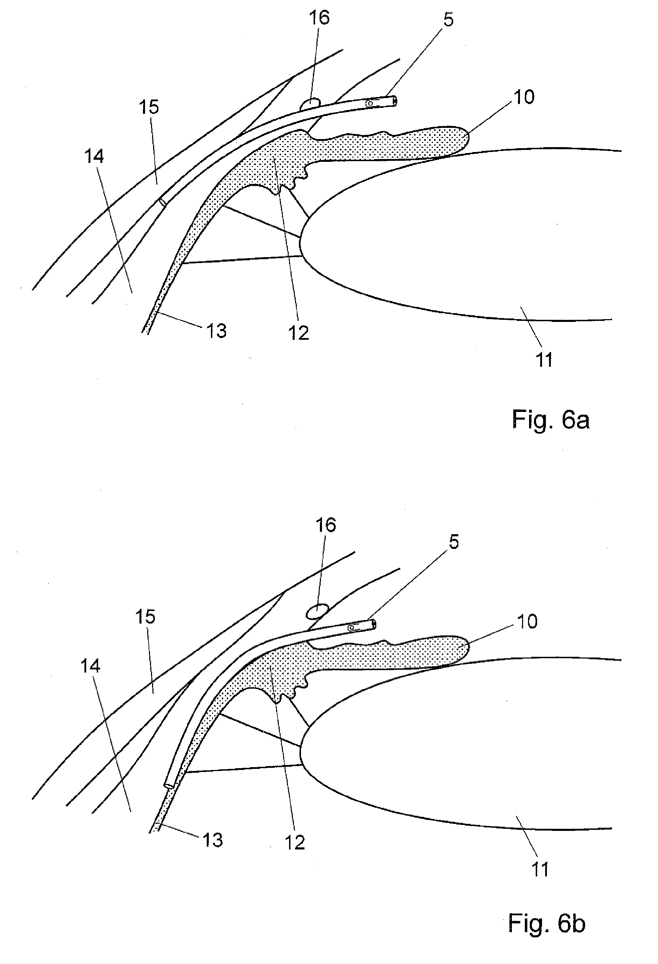

- Figur 6 a): eine schematische Darstellung der bevorzugten Positionierung des erfindungsgemäßen Augenimplantats zur Drainage des Kammerwassers in den Subkonjunktivalraum, und
- Figur 6 b): eine schematische Darstellung der bevorzugten Positionierung des erfindungsgemäßen Augenimplantats zur Drainage des Kammerwassers in den Uveoskleralraum.

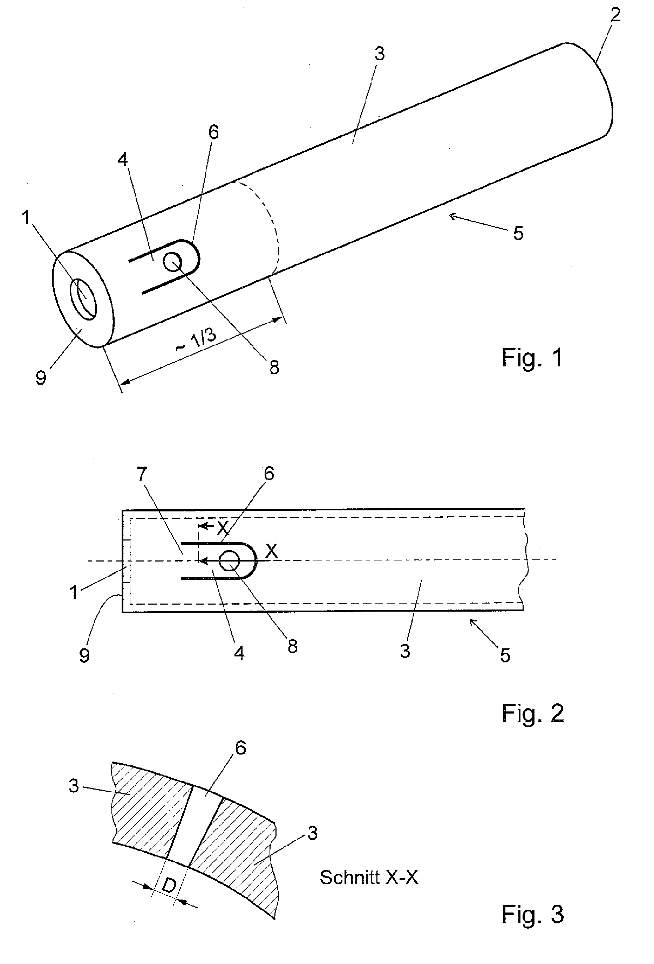

Figur 1 zeigt einen erfindungsgemäßen Stent (Augenimplantat) in perspektivischer Darstellung. Der Stent besteht aus einem Röhrchen 5, das vorzugsweise einen Innendurchmesser von 350 bis 400 µm aufweist. Dieser Innendurchmesser ist derart bemessen, dass sich der Flusswiderstand des im Röhrchen 5 gebildeten Hohlkanals nicht wesentlich ändert, da Phänomene, wie beispielsweise Fibrose o.ä., die ein Zusetzen von Öffnungen hervorrufen können, bei einem solch großen Innendurchmesser keine bzw. vernachlässigbar geringe Auswirkungen haben. Der durch das Röhrchen 5 gebildete Hohlkanal weist eine erste Öffnung 1 sowie eine zweite Öffnung 2 auf. Der Stent 5 wird später derart im Auge platziert, dass sich die erste Öffnung 1 in der vorderen Augenkammer und die zweite Öffnung 2 außerhalb der vorderen Augenkammer, beispielsweise im Subkonjunktivalraum, oder im Uveoskleralraum, befinden. Dabei ist es vorzugsweise vorgesehen, dass sich - von der ersten Öffnung 1 ausgehend - ca. ein Drittel der Länge des Stents 5 (entlang seiner Längsachse) in der vorderen Augenkammer befindet. Weiterhin ist es erfindungsgemäß vorgesehen, dass eine Durchflussbegrenzung, welche vorzugsweise als Blende 9 ausgebildet und direkt an der ersten Öffnung 1 angeordnet ist, vorgesehen wird. Der Durchmesser der Blende 9 wird je nach Augeninnendruck des Patienten bemessen und rangiert vorzugsweise in einem Bereich zwischen 30 und 70 µm. Dieser Durchmesser muss derart bemessen sein, dass eine Hypotonie des Auges, welche zu einem Einfall der vorderen Augenkammer führen könnte, sicher außergeschlossen wird.

Auf der Wandfläche (Mantelfläche) 3 des Stents 5 befindet sich erfindungsgemäß mindestens ein druckgesteuertes Ventil 4, welches sich ab einem Augeninnendruck von vorzugsweise 10 mm Hg-Säule kontinuierlich (bis zu einem Augeninnendruck von vorzugsweise 20 mm Hg-Säule) öffnet. Durch ein solches druckgesteuertes Ventil 4 wird eine zusätzliche Anströmmöglichkeit des Augenkammerwassers in den Stent 5 und damit eine zusätzliche Abflussmöglichkeit für das Augenkammerwasser bei erhöhtem Druck geschaffen, selbst wenn der Durchflusswiderstand durch die Blende 9 - beispielsweise durch Fibrose im Laufe der Zeit - erhöht wird. Dadurch kann eine Druckregulierung auch für lange Zeit gewährleistet werden, so dass der erfindungsgemäße Stent 5 bei einem Patienten länger als die herkömmlichen Stents nach dem Stand der Technik im Auge verbleiben kann. Dadurch können zusätzliche Operationen zur Auswechslung eines Stents vermieden werden.

Figur 2 zeigt den in Figur 1 dargestellten Stent in Aufsicht. Das druckgesteuerte Ventil 4 ist vorzugsweise als Zungenmembranventil ausgestaltet. Ein solches Zungenmembranventil 4 weist eine Basis 7 sowie eine umlaufende Durchtrennung 6 der Wandfläche 3 auf. Die umlaufende Durchtrennung 6 ermöglicht es, dass das druckgesteuerte Ventil 4 bei erhöhtem Augenkammerwasserdruck nach innen (in Richtung des Hohlkanals) ausweicht und somit eine zusätzliche Zuflussmöglichkeit für das Augenkammerwasser gebildet wird. Das druckgesteuerte Ventil 4 kann vorzugsweise als Zungenmembranventil, jedoch auch durch andere Ausgestaltungen gebildet werden. Dadurch ist es vorzugsweise vorgesehen, dass eine partiell umlaufende Durchtrennung eine Öffnung des Ventils bei erhöhtem Augeninnendruck (vorzugsweise ab 10 mm Hg-Säule) ermöglicht.

Figur 3 zeigt eine vergrößerte Schnittdarstellung des in Figur 2 gezeigten Augenimplantats entlang der Linie X-X'. Es ist ersichtlich, dass die (partiell) umlaufende Durchtrennung 6 durch einen Spalt in der Wandfläche 3 ausgebildet wird. Dieser Spalt weist vorzugsweise eine Spaltbreite D zwischen 10 und 20 µm auf. Bei einer solchen Spaltbreite kommt dem Spalt selbst keine Strömungsfunktion zu; das bedeutet, dass das Augenkammerwasser selbst durch den Spalt nicht ablaufen kann, sofern das druckgesteuerte Ventil 4 nicht durch Biegung in Richtung des Hohlkanals geöffnet wird. Der Spalt sichert die freie Beweglichkeit der Ventilzunge. In einer bevorzugten Ausgestaltung ist der Spalt konisch ausgebildet.

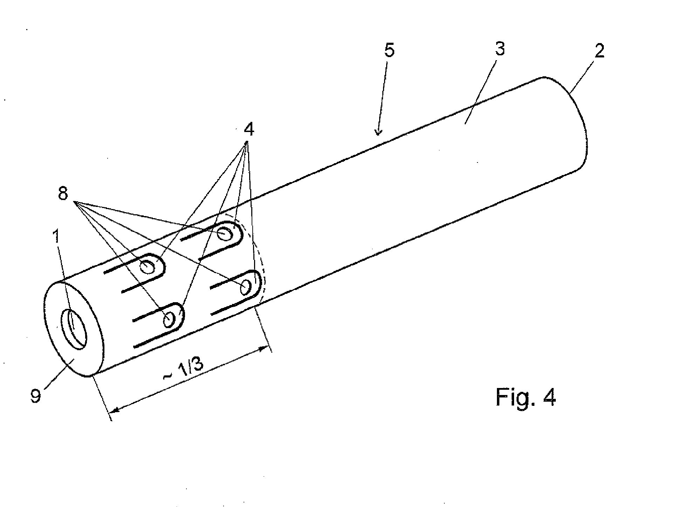

Figur 4 zeigt einen erfindungsgemäßen Stent in perspektivischer Darstellung, der eine Vielzahl von druckgesteuerten Ventilen 4 aufweist. Um den zusätzlich möglichen Anströmbereich des Stents innerhalb der vorderen Augenkammer möglichst groß zu halten, und um gleichzeitig eine möglichst hohe und ausreichende Stabilität des gesamten Stents zu gewährleisten, werden vorzugsweise eine Vielzahl von druckgesteuerten Ventilen 4, die jedoch nicht mehr als 30 % der Gesamtmanteloberfläche des Stents bedecken, vorgesehen. In einer besonders bevorzugten Ausführungsvariante sind je vier Ventile umlaufend versetzt in je zwei Reihen hintereinander (entlang der Längsachse des Stents) vorgesehen.

Figur 5 a) zeigt ein erfindungsgemäßes druckgesteuertes Ventil 4 in schematischer, geschnittener Darstellung. Das druckgesteuerte Ventil 4 wird durch eine, in der Wandfläche 3 vorgesehene, partiell umlaufende Durchtrennung 6 der Wandfläche 3 ausgebildet. Lediglich im Bereich der Basis 7 des druckgesteuerten Zungenmembranventils 4 ist die Wandfläche 3 nicht vollständig durchtrennt. Figur 5 c) zeigt das erfindungsgemäße druckgesteuerte Ventil 4 in geöffnetem Zustand, wobei das entsprechende druckgesteuerte Ventil 4 in Figur 5 b) in geschlossenem Zustand schematisch dargestellt ist. Wie aus den Figuren 5b) und c) ersichtlich, ist die Dicke der Wandfläche 3 im Bereich der Basis 7 verringert, so dass das druckgesteuerte Ventil 4 in einfacher Weise in Richtung des Hohlkanals federnd öffnen kann, sofern ein erhöhter Augeninnendruck in der vorderen Augenkammer vorhanden sein sollte.

Wie in den Figuren 1, 2, 4 und 5a) ersichtlich ist, können die druckgesteuerten Ventile 4 im Bereich ihrer Membran (Zunge) zusätzliche Öffnungen 8 aufweisen, die einen weiteren (dauerhaften) Abfluss des Augenkammerwassers ermöglichen.

Die Figuren 6 a) und 6b) zeigen beispielhaft eine mögliche Platzierung des Augenimplantats mit Lage der Öffnung 1 in der vorderen Augenkammer und der Öffnung 2 im Subkonjunktivalraum (Figur 6 a) oder im Uveoskleralraum (Figur 6b).

### Bezugszeichenliste

- 1: erste Öffnung
- 2: zweite Öffnung
- 3: Wandfläche
- 4: druckgesteuertes Ventil
- 5: Röhrchen/Augenimplantat
- 6: umlaufende Durchtrennung der Wandfläche/Spalt
- 7: Zungenbasis/Zungenbein
- 8: Öffnung im Ventil
- 9: Blende
- 10: Iris
- 11: Linse
- 12: Ziliarkörper
- 13: Aderhaut
- 14: Sklera
- 15: Bindehaut
- 16: Trabekelmaschenwerk/ Schlemmscher Kanal

## Patentansprüche

1. Augenimplantat, aufweisend:
ein Röhrchen (5), dessen Wandfläche (3) einen Hohlkanal einschließt, der beidseitig in Längserstreckung des Hohlkanals offen ausgebildet ist, wobei eine erste Öffnung (1) zum Einströmen von Augenkammerwasser und eine zweite Öffnung (2) zum Abfließen des Augenkammerwassers vorgesehen ist, und wobei die Wandfläche (3) durch ein flüssigkeitsdichtes Material ausgebildet ist, wobei im Bereich der Wandfläche (3) mindestens ein druckgesteuertes Ventil (4) angeordnet ist, **dadurch gekennzeichnet, dass**
das druckgesteuerte Ventil (4) ein Membranventil (4) ist, welches durch eine Basis (7) und eine umlaufende Durchtrennung (6) der Wandfläche (3) ausgebildet ist, wobei die Wandfläche (3) im Bereich der Basis (7) nicht durchtrennt ist.

2. Augenimplantat nach Anspruch 1,
**dadurch gekennzeichnet, dass**
das Wandmaterial und die Wandstärke des Röhrchens (5) derart gewählt sind, dass das Röhrchen (5) nach Einbringen in die Vorderkammer des Auges derart in dieser platziert verbleibt, dass sich die zweite Öffnung (2) im Subkonjunktivalraum oder im Uveoskeralraum befindet.

3. Augenimplantat nach einem der vorhergehenden Ansprüche,
**dadurch gekennzeichnet, dass**
die Wandfläche (3) des Röhrchens (5) aus einem flexiblen Material, einem Metall oder einer Metalllegierung, bevorzugt einem Elastomer, einer Formgedächtnislegierung (NiTi) oder dem Edelstahl 316L ausgebildet ist.

4. Augenimplantat nach einem der vorhergehenden Ansprüche,
**dadurch gekennzeichnet, dass**
das Membranventil (4) als Zungenmembranventil (4) ausgebildet ist.

5. Augenimplantat nach Anspruch 4,
**dadurch gekennzeichnet, dass**
dass Membranventil (4) durch die Basis (7) und eine zungenförmig umlaufende Durchtrennung (6) der Wandfläche (3) ausgebildet ist, wobei die Wandfläche (3) im Bereich der Basis (7) nicht durchtrennt ist.

6. Augenimplantat nach einem der Ansprüche 4 und 5,
**dadurch gekennzeichnet, dass**
die Wandstärke der Wandfläche (3) im Bereich der Basis (7) geringer ist als die Wandstärke der Wandfläche (3) im angrenzenden Bereich außerhalb des Membranventils (4).

7. Augenimplantat nach Anspruch 6,
**dadurch gekennzeichnet, dass**
die Wandstärke der Wandfläche (3) im Bereich der Basis (7) zwischen 30 und 80 % der Wandstärke der Wandfläche (3) im angrenzenden Bereich außerhalb des Membranventils (4) beträgt.

8. Augenimplantat nach einem der Ansprüche 1 und 7,
**dadurch gekennzeichnet, dass**
die Basis (7) des Membranventils (4) zur ersten Öffnung (1) ausgerichtet und die umlaufende Durchtrennung (6) von der Basis (7) in Richtung der zweiten Öffnung (2) ausgerichtet ist.

9. Augenimplantat nach Anspruch 1,
**dadurch gekennzeichnet, dass**
das Membranventil (4) eine Fläche aufweist, die zwischen 5 % und 10 % der Fläche der Wandfläche (3) des Röhrchens (5) entspricht.

10. Augenimplantat nach Anspruch 1,
**dadurch gekennzeichnet, dass**
das Membranventil (4) eine axiale Längsausdehnung entlang der Längsachse des Röhrchens (5) von maximal 15 % der Länge des Röhrchens (5) und eine radiale Querausdehnung von maximal 15 % der Umfanges des Röhrchens (5) aufweist.

11. Augenimplantat nach Anspruch 1,
**dadurch gekennzeichnet, dass**
eine Vielzahl von Membranventilen (4), vorzugsweise 2 bis 8 Membranventile vorgesehen sind.

12. Augenimplantat nach einem der vorhergehenden Ansprüche,
**dadurch gekennzeichnet, dass**
der Hohlkanal eine durch eine Blende (9) ausgebildete Flussbegrenzung aufweist, wobei die Blende (9) eine Öffnung aufweist, deren Fläche zwischen 1000 und 3000 µm², bevorzugt zwischen 1500 und 2000 µm² entspricht.

13. Augenimplantat nach Anspruch 13,
**dadurch gekennzeichnet, dass**
die Blende (9) in einem Abstand von der ersten Öffnung (1) angeordnet ist, der zwischen 0 % und 10 % der Längserstreckung des Röhrchens (5) entspricht.

14. Augenimplantat nach Anspruch 1,
**dadurch gekennzeichnet, dass**
das mindestens eine druckgesteuerte Membranventil (4) derart ausgebildet ist, dass es zwischen einem Druck von 10 mm bis 20 mm Hg-Säule kontinuierlich öffnet.

15. Augenimplantat nach Anspruch 1,
**dadurch gekennzeichnet, dass**
das druckgesteuerte Membranventil (4) in einem Abstand von der ersten Öffnung (1) angeordnet ist, der zwischen 0 % und 35 % der Längserstreckung des Röhrchens (5) entspricht.

## Claims

1. An eye implant, comprising
a small tube (5), the wall surface (3) of which includes a hollow channel which is open at both sides in the longitudinal direction of the hollow channel, wherein a first opening (1) for the inflow of aqueous humour and a second opening (2) for the outflow of aqueous humour is provided, and wherein the wall surface (3) is made of a fluid-impervious material, whereby in the region of the wall surface (3) at least one pressure-controlled valve (4) is arranged,
**characterised in that**
the pressure-controlled valve (4) is a membrane valve (4) which is formed by a base (7) and a circumferential transection (6) of the wall surface (3), wherein the wall surface (3) is not transected in the region of the base (7).

2. The eye implant according to claim 1,
**characterised in that**
the wall material and the wall thickness of the small tube (5) are selected in such a way that after having been inserted into the anterior chamber of the eye, the small tube (5) remains placed therein in such a way that the second opening (2) is located in the subconjunctival space or in the uveoscleral space.

3. The eye implant according to any one of the preceding claims,
**characterised in that**
the wall surface (3) of the small tube (5) is made of a flexible material, a metal or a metal alloy, preferably an elastomer, a shape-memory alloy (NiTi) or stainless steel 316L.

4. The eye implant according to any one of the preceding claims,
**characterised in that**
the membrane valve (4) is designed as a tongue membrane valve (4).

5. The eye implant according to claim 4,
**characterised in that**
the membrane valve (4) is formed by the base (7) and a tongue-shaped circumferential transection (6) of the wall surface (3), wherein the wall surface (3) is not transected in the region of the base (7).

6. The eye implant according to any one of claims 4 and 5,
**characterised in that**
the wall thickness of the wall surface (3) in the region of the base (7) is less than the wall thickness of the wall surface (3) in the adjacent area outside the membrane valve (4).

7. The eye implant according to claim 6,
**characterised in that**
the wall thickness of the wall surface (3) in the region of the base (7) is between 30 and 80 % of the wall thickness of the wall surface (3) in the adjacent area outside the membrane valve (4).

8. The eye implant according to one of claims 1 and 7,
**characterised in that**
the base (7) of the membrane valve (4) is aligned in relation to the first opening (1) and the circumferential transection (6) of the base (7) is aligned in relation to the second opening (2).

9. The eye implant according to claim 1,
**characterised in that**
the membrane valve (4) has a surface area that corresponds to between 5 % and 10 % of the area of the wall surface (3) of the small tube (5).

10. The eye implant according to claim 1,
**characterised in that**
the membrane valve (4) has an axial longitudinal dimension along the longitudinal axis of the small tube (5) of max. 15 % of the length of the small tube (5) and a transverse dimension of max. 15 % of the circumference of the small tube (5).

11. The eye implant according to claim 1,
**characterised in that**
a plurality of membrane valves (4), preferably 2 to 8 membrane valves are envisaged.

12. The eye implant according to any one of the preceding claims,
**characterised in that**
the hollow channel has a flow limiter formed by a diaphragm (9), wherein the diaphragm (9) has an aperture with an area of between 1000 and 3000 µm², preferably between 1500 and 2000 µm².

13. The eye implant according to claim 13,
**characterised in that**
the diaphragm (9) is arranged at a distance from the first opening (1) which corresponds to between 0 % and 10 % of the longitudinal dimension of the small tube (5).

14. The eye implant according to claim 1,
**characterised in that**
at least one pressure-controlled membrane valve (4) is designed in such a way that it opens continuously at a pressure of between 10 mm and 20 mm Hg column.

15. The eye implant according to claim 1,
**characterised in that**
the pressure-controlled membrane valve (4) is arranged at a distance from the first opening (1) which corresponds to between 0 % and 35 % of the longitudinal dimension of the small tube (5).

## Revendications

1. Implant oculaire présentant :
une tubule (5) dont la surface de paroi (3) circonscrit un canal creux qui a une conformation ouverte des deux côtés dans l'extension longitudinale du canal creux, dans lequel une première ouverture (1) est prévue pour l'afflux de liquide de la chambre de l'oeil et une seconde ouverture (2) pour l'écoulement de liquide de la chambre de l'oeil et la surface de paroi (3) est constituée d'un matériau étanche au liquide, au moins une soupape commandée par pression (4) étant disposée au niveau de la surface de paroi (3), **caractérisé en ce que**
la soupape commandée par pression (4) est une soupape à membrane (4) qui est constituée d'une base (7) et d'une cloison périphérique (6) de la surface de paroi (3), la surface de paroi (3) n'étant pas cloisonnée au niveau de la base (7).

2. Implant oculaire selon la revendication 1,
**caractérisé en ce que**
le matériau de paroi et l'épaisseur de paroi de la tubule (5) sont sélectionnés de manière à ce que la tubule (5), une fois introduite dans la chambre antérieure de l'oeil, reste placée dans celle-ci de telle sorte que la seconde ouverture (2) se trouve dans l'espace sous-conjonctival ou dans l'espace uvéoscléral.

3. Implant oculaire selon une des revendications précédentes,
**caractérisé en ce que**
la surface de paroi (3) de la tubule (5) est composée d'un matériau souple, d'un métal ou d'un alliage métallique, de préférence un élastomère, un alliage à mémoire de forme (NiTi) ou l'acier inoxydable 316L.

4. Implant oculaire selon une des revendications précédentes,
**caractérisé en ce que**
la soupape à membrane (4) se présente sous forme d'une soupape à membrane à languette (4).

5. Implant oculaire selon la revendication 4,
**caractérisé en ce que**
la soupape à membrane (4) est constituée par la base (7) et une cloison périphérique en forme de languette (6) de la surface de paroi (3), la surface de paroi (3) n'étant pas cloisonnée au niveau de la base (7).

6. Implant oculaire selon une des revendications 4 et 5,
**caractérisé en ce que**
l'épaisseur de paroi de la surface de paroi (3) est plus faible au niveau de la base (7) que l'épaisseur de paroi de la surface de paroi (3) dans la zone adjacente à l'extérieur de la soupape à membrane (4).

7. Implant oculaire selon la revendication 6,
**caractérisé en ce que**
l'épaisseur de paroi de la surface de paroi (3) au niveau de la base (7) représente 30 à 80 % de l'épaisseur de paroi de la surface de paroi (3) dans la zone adjacente à l'extérieur de la soupape à membrane (4).

8. Implant oculaire selon une des revendications 1 et 7,
**caractérisé en ce que**
la base (7) de la soupape à membrane (4) est orientée vers la première ouverture (1) et la cloison périphérique (6) de la base (7) en direction de la seconde ouverture (2).

9. Implant oculaire selon la revendication 1,
**caractérisé en ce que**
la soupape à membrane (4) a une surface qui représente de 5 % à 10 % de la surface de paroi (3) de la tubule (5).

10. Implant oculaire selon la revendication 1,
**caractérisé en ce que**
la soupape à membrane (4) présente une extension longitudinale le long de l'axe longitudinal de la tubule (5) représentant au maximum 15 % de la longueur de la tubule (5) et une extension transversale représentant au maximum 15 % de la circonférence de la tubule (5).

11. Implant oculaire selon la revendication 1,
**caractérisé en ce**
**qu'**il est prévu une multitude de soupapes à membrane (4), de préférence 2 à 8 soupapes à membrane.

12. Implant oculaire selon une des revendications précédentes,
**caractérisé en ce que**
le canal creux présente un limiteur de flux constitué par un écran (9), l'écran (9) présentant une ouverture dont la surface est de 1000 à 3000 µm², de préférence 1500 à 2000 µm².

13. Implant oculaire selon la revendication 13,
**caractérisé en ce que**
l'écran (9) est disposé à une distance de la première ouverture (1) qui représente de 0 % à 10 % de l'extension longitudinale de la tubule (5).

14. Implant oculaire selon la revendication 1,
**caractérisé en ce que**
l'au moins une soupape à membrane contrôlée par pression (4) est réalisée de manière à s'ouvrir en continu entre une pression de 10 mm à 20 mm de colonne de Hg.

15. Implant oculaire selon la revendication 1,
**caractérisé en ce que**
la soupape à membrane contrôlée par pression (4) est disposée à une distance de la première ouverture (1) qui représente 0 % à 35 % de l'extension longitudinale de la tubule (5).
